# EUROPEAN PATENT APPLICATION

(11) **EP 2 886 152 A1**
(43) Date of publication of application: **24.06.2015**
(21) Application number: 13828705.7
(22) Date of filing: 15.07.2013
(51) Int. Cl.: A61M 39/08, C08L 53/02

(54) **POLYVINYL CHLORIDE-FREE MEDICAL TUBE, AND MOLDED ARTICLES AND MEDICAL SUPPLIES PRODUCED THEREWITH**

(30) Priority: 07.08.2012 KR 20120086047; 07.08.2012 KR 20120086079
(71) Applicant: Polyscientech Inc., Bogae-myeon, Anseong-si Gyeonggi-do 456-874 (KR)
(72) Inventor: JEON, Seung Ho, Gyeonggi-do 448-172 (KR); PARK, Chang Kyu, Seoul 130-020 (KR); PARK, Jong, Gyeonggi-do 446-724 (KR)
(74) Representative: Stolmár & Partner Patentanwälte PartG mbB
(86) International application number: PCT/KR2013/006313
(87) International publication number: WO 2014/025142

(57) **Abstract**

Provided is a medical tube including at least one layer made of a composition containing a cross-copolymerized olefin-aromatic vinyl compound-diene copolymer. The medical tube according to the present invention, which has excellent kink resistance, toughness, transparency, non-adsorption of drugs, and the like, and is made of a non-toxic polyvinyl chloride (PVC)-free material, may be combined with an injection-molded article of the resin, the resultant article may be effectively used for medical supplies such as an infusion set, a blood transfusion set, a medical liquid container set, a medical catheter, and the like.

## Description

### [Technical Field]

The present invention relates to a polyvinyl chloride- free (PVC-free) medical tube and molded articles and medical supplies produced therewith. More particularly, the present invention relates to a medical tube made of a non-toxic PVC-free material, having excellent kink resistance, toughness, transparency, and non-adsorption of drugs, etc. and molded articles and medical supplies produced therewith.

### [Background Art]

According to the related art, as a medical tub, a tube molded using flexible polyvinyl chloride (PVC) containing a large amount of a plasticizer such as diethylhexylphthalate, or the like, has been usefully used in medical supplies such as a connection line configuring an infusion set, a connection line configuring a blood transfusion set, a port of a medical liquid container, a medical catheter, or the like due to advantages such as excellent kink resistance, toughness, and transparency, etc.

However, in the case of infusing a drug such as nitroglycerin, a tranquilizer such as diazepam, or the like, a flexible PVC tube has a problem in that the drug may be adsorbed in the tube. In addition, in the case of infusing a drug including a surfactant as a solubilizing agent of a drug, the plasticizer is eluted from the tube, which causes serious problems, for example, reaction of an so-called environmental hormone causing human endocrine disruption, or the like, and at the time of incinerating PVC, there is a risk that dioxin, which is a representative environmental hormone, will be generated, such that development of medical supplies composed of a tube made using a so-called PVC-free material, or the like, capable of replacing the flexible PVC has been urgently required.

As a method for solving the problem, a PVC-free tube using a composition of polypropylene and a polyethylene based elastomer or hydrogenated styrene-diene elastomer has been suggested in Japanese Patent Laid-Open Publication No. 2003-205033. In this case, non-adsorption of drugs is excellent, but kink resistance is significantly poor, and tensile strength is satisfactory to some degree, but flexibility is significantly insufficient, and as a result, toughness is insufficient, such that the PVC-free tube did not solve the problem.

Generally, toughness is evaluated as tensile strength and elongation at the time of fracture by measuring tensile properties of a tube, and in the case in which elongation is large, flexibility is excellent, and in the case in which tensile strength and elongation are simultaneously excellent, toughness is excellent. As the toughness suitable for a medical tube, a tube should have tensile strength of 15MPa or more, preferably, 20Mpa or more, and simultaneously have elongation of 600% or more, preferably 700% or more.

As another solution, a PVC-free tube using syndiotactic 1,2-polybutadiene having a crystallinity of 5% or more has been suggested in Japanese Patent Laid-Open Publication No. 2004-321788. In this case, kink resistance, transparency, and non-adsorption of drugs are excellent, but flexibility is somewhat insufficient, while tensile strength is significantly insufficient, such that overall, toughness is insufficient.

As another solution, a PVC-free tube made of a composition of isotactic polypropylene and a polypropylene based elastomer has been suggested in Korean Patent No. 10-0909393. In this case, transparency and non-adsorption of drugs are excellent, but kink resistance is insufficient, and tensile strength is excellent but flexibility is insufficient, such that toughness is insufficient.

Meanwhile, a method of solving the problem using a tube having a multilayer structure rather than a tube having a single layer structure has also been suggested. For example, a PVC-free tube having a multilayer structure of two or three layers composed of a syndiotactic 1,2-polybutadiene layer and a polyethylene layer made of linear low density polyethylene, or the like, has been suggested in Japanese Patent Laid-Open Publication No. 2007-236781. In this case, tensile strength is slightly improved as compared to a tube having a single layer structure made of only syndiotactic 1,2-polybutadiene, but is still insufficient, and elongation is rather deteriorated, such that overall, toughness is insufficient, and kink resistance is somewhat insufficient. Therefore, these problems should be solved.

Further, in the case of medical supplies mainly composed of a tube such as the infusion set, the blood transfusion set, a medical liquid container set, the medical catheter, or the like, in order to complete perfect PVC-free medical supplies, generally injection-molded articles, for example, in the case of the infusion set, a drip chamber should also be developed as a PVC-free product, and a measure for the PVC-free product has been required.

### [Related Art Document]

Japanese Patent Laid-Open Publication No. 2003-205033 (July 22, 2003)
Japanese Patent Laid-Open Publication No. 2004-321788 (November 18, 2004)
Korean Patent No. 10-0909398 (July 20, 2009)
Japanese Patent Laid-Open Publication No. 2007-236781 (September 20, 2007)

### [Disclosure]

### [Technical Problem]

The present inventors conducted studies in order to solve the above-mentioned problems, thereby completing the present invention. An object of the present invention is to provide a medical tube made of a non-toxic PVC-free material, having excellent kink resistance, toughness, transparency, and non-adsorption of drugs, etc., and molded articles and medical supplies produced therewith.

### [Technical Solution]

In one general aspect, a medical tube includes at least one layer, made of a cross-copolymerized olefin-aromatic vinyl compound-diene copolymer having Shore hardness of 40A or more but 100A or less, or a resin composition containing the copolymer. The cross-copolymerized olefin-aromatic vinyl compound-diene copolymer may be obtained by cross-copolymerizing a monomer containing at least 5 mol% of an aromatic vinyl compound with an olefin-aromatic vinyl compound-diene copolymer in which an aromatic vinyl compound is contained in a range of 0.03 mol% or more but 96 mol% or less, diene is contained in a range of 0.0001 mol% or more but 3 mol% or less, and the remainder is olefin. In addition, a melt index(g/10min) of the cross-copolymerized olefin-aromatic vinyl compound-diene copolymer measured at 200°C under a load of 10 kg may be 1 to 50.

The resin composition may contain 10 to 200 parts by weight of syndiotactic 1,2-polybutadiene, a polyethylene based elastomer, or a mixture thereof, based on 100 parts by weight of the cross-copolymerized olefin-aromatic vinyl compound-diene copolymer having Shore hardness of 40A or more but 100A or less.

Alternatively, the resin composition may contain 2 to 30 parts by weight of the cross-copolymerized olefin-aromatic vinyl compound-diene copolymer having Shore hardness of 40A or more but 100A or less, based on 100 parts by weight of a polypropylene based elastomer having Shore hardness of 35A or more but 100A or less.

Alternatively, the resin composition may contain 2 to 30 parts by weight of the cross-copolymerized olefin-aromatic vinyl compound-diene copolymer having Shore hardness of 40A or more but 100A or less and 5 to 50 parts by weight of any one selected from polypropylene resins and styrene based elastomers or a mixture thereof, based on 100 parts by weight of the polypropylene based elastomer having Shore hardness of 35A or more but 100A or less.

The medical tube may be a tube having a two layer structure composed of one layer made of the cross-copolymerized olefin-aromatic vinyl compound-diene copolymer having Shore hardness of 40A or more but 100A or less or the resin composition containing the copolymer and the other layer made of syndiotactic 1,2-polybutadiene, the polyethylene based elastomer, or the mixture thereof.

The medical tube may be a tube having a three layer structure composed of an intermediate layer made of the cross-copolymerized olefin-aromatic vinyl compound-diene copolymer having Shore hardness of 40A or more but 100A or less or the resin composition containing the copolymer, and internal and external layers made of syndiotactic 1,2-polybutadiene, the polyethylene based elastomer, or the mixture thereof.

The medical tube may be a tube having a three layer structure composed of internal and external layers made of the cross-copolymerized olefin-aromatic vinyl compound-diene copolymer having Shore hardness of 40A or more but 100A or less or the resin composition containing the copolymer, and an intermediate layer made of syndiotactic 1,2-polybutadiene, the polyethylene based elastomer, or the mixture thereof.

The medical tube may be a tube having a two layer structure composed of one layer made of a resin composition containing 5 to 50 parts by weight of a polypropylene resin and 2 to 30 parts by weight of the cross-copolymerized olefin-aromatic vinyl compound-diene copolymer having Shore hardness of 40A or more but 100A or less, based on 100 parts by weight of the polypropylene based elastomer having Shore hardness of 35A or more but 100A or less and the other layer made of the polypropylene based elastomer having Shore hardness of 35A or more but 100A or less.

The medical tube may be a tube having a three layer structure composed of an intermediate layer made of a resin composition containing 5 to 50 parts by weight of a polypropylene resin or styrene based elastomer and 2 to 30 parts by weight of the cross-copolymerized olefin-aromatic vinyl compound-diene copolymer having Shore hardness of 40A or more but 100A or less, based on 100 parts by weight of the polypropylene based elastomer having Shore hardness of 35A or more but 100A or less and internal and external layers made of the polypropylene based elastomer having Shore hardness of 35A or more but 100A or less.

The medical tube may be a tube having a three layer structure composed of internal and external layers made of a resin composition containing 5 to 50 parts by weight of a polypropylene resin or styrene based elastomer and 2 to 30 parts by weight of the cross-copolymerized olefin-aromatic vinyl compound-diene copolymer having Shore hardness of 40A or more but 100A or less, based on 100 parts by weight of the polypropylene based elastomer having Shore hardness of 35A or more but 100A or less and an intermediate layer made of the polypropylene based elastomer having Shore hardness of 35A or more but 100A or less.

In another general aspect, a medical supply includes the medical tube as described above, wherein the medical supply is any one of an infusion set, a medical connection line, a port of a medical liquid container, and a medical catheter.

In another general aspect, there is provided a medical molded article manufactured by injection-molding a cross-copolymerized olefin-aromatic vinyl compound-diene copolymer having Shore hardness of 40A or more but 100A or less or a composition obtained by adding 10 to 200 parts by weight of syndiotactic 1,2-polybutadiene, a polyethylene based elastomer, or a mixture thereof, based on 100 parts by weight of the cross-copolymerized olefin-aromatic vinyl compound-diene copolymer having Shore hardness of 40A or more but 100A or less.

An object of the present invention is to develop a medical tube made of a material simultaneously having excellent kink resistance, toughness, transparency, and non-adsorption of drugs, etc, as a PVC-free material. As problems in the related art, in most cases, flexibility was excellent but tensile strength was insufficient, or on the contrary, tensile strength was excellent but flexibility was insufficient.

The present inventors conducted research and development using various synthetic resins having the above-mentioned characteristics based on an idea that since a general drug is significantly hydrophilic, in the case of using a material having a lipophilic property, a flexibility of 600% or more, preferably 700% or more (based on elongation at the time of performing a tensile test), and a strength of 15MPa or more, preferably 20MPa or more (based on tensile strength) as the PVC-free material, the desired object may be achieved. While conducting the researches, the present inventors found that in the case of mainly using the cross-copolymerized olefin-aromatic vinyl compound-diene copolymer having Shore hardness of 40A or more but 100A or less or in the case of adding a suitable amount of the cross-copolymerized olefin-aromatic vinyl compound-diene copolymer to a mixture of a polypropylene based elastomer having Shore hardness of 35A or more but 100A or less and a polypropylene resin or a mixture of a polypropylene based elastomer having Shore hardness of 35A or more but 100A or less and a styrene based elastomer, all of the desired characteristics were expressed, thereby completing the present invention.

The reason may be that in the case of using a mixture of a polypropylene based elastomer and a polypropylene resin according to related art, kink resistance was significantly poor, in the case of using a mixture of a polypropylene based elastomer and a styrene based elastomer, toughness, particularly, rigidity was severely weak, but the suitably added cross-copolymerized olefin-aromatic vinyl compound-diene copolymer acted as a kind of significantly excellent polymer based compatibilizer at a molecular level between the polypropylene based elastomer and the polypropylene resin or styrene based elastomer to thereby express a morphology at a nanoalloy level and have synergic effects in view of physical properties, such that excellent transparency as well as kink resistance and toughness were simultaneously secured.

In the present invention, the cross-copolymerized olefin-aromatic vinyl-diene copolymer is a copolymer prepared by copolymerizing monomers including at least 5 mol% of a aromatic vinyl monomer with the olefin-aromatic vinyl compound-diene copolymer containing an aromatic vinyl compound such as styrene, p-chlorostyrene, p-tert-butylstyrene, α-methylstyrene, p-methylstyrene, vinylnaphthalene, vinylanthracene, or the like in a range of 0.03 mol% or more but 96 mol% or less, diene, which is any one or a mixture of at least two of ortho-divinylbenzene, para-divinylbenzene, and meta -divinylbenzene, in a range of 0.0001 mol% or more but 3 mol% or less, and the remainder being ethylene or at least two kinds of α-olefins including ethylene and α-olefin having 3 to 20 carbon atoms such as propene, 1-butene, 4-methyl-pentene-1, hexene, octene, decene, and the melt index thereof measured at 200°C and under a load of 10 kg is 1 to 50 (g/10min), preferably, 1 to 30 (g/10min). This resin may be prepared by methods disclosed in U.S. Patent No. 6,559,234, U.S. Patent No. 6,566,453, Japanese Patent Laid-Open Publication No. 2002-003555, and Japanese Patent Laid-Open Publication No. 2009-299068, and as a more specific example, there are SE based flexible resins (Japanese Electro-Chemical Industry Co.), Grade AC25, AC081, AC095, AC097, AC098, ACP2520, and the like.

Further, in view of the object of the present invention, it is preferable that the cross-copolymerized olefin-aromatic vinyl compound-diene copolymer has Shore hardness of 40A or more but 100A or less, preferably 50A or more but 90A or less. In the case in which the Shore hardness is less than 40A, the copolymer is excessively flexible, such that it is difficult to secure the desired rigidity, and in the case in which the Shore hardness is more than 100A, flexibility is insufficient, such that it is also difficult to secure the desired rigidity. Further, due to the same reason, the composition containing the cross-copolymerized olefin-aromatic vinyl compound-diene copolymer has Shore hardness of 40A or more but 100A or less, which is preferable in view of obtaining a product having excellent rigidity.

In the medical tube according to the present invention, a mixture of the cross-copolymerized olefin-aromatic vinyl compound-diene copolymer having Shore hardness of 40A or more but 100A or less and syndiotactic 1,2-polybutadiene or the polyethylene based elastomer in addition to the copolymer is used, which is preferable in that a product having excellent flexibility and elastic recovery rate may be produced. In the case of using the mixture, it is preferable that a composition ratio of the added syndiotactic 1,2-polybutadiene, the polyethylene based elastomer, or the mixture thereof is 10 to 200 parts by weight, preferably, 20 to 150 parts by weight, based on 100 parts by weight of the cross-copolymerized olefin-aromatic vinyl compound-diene copolymer having Shore hardness of 40A or more but 100A or less. In the case of the composition ratio, a medical tube of which toughness is increased, physical properties are excellent, and elastic modulus and flexibility are not deteriorated may be provided.

In the present invention, the polyethylene based elastomer is an elastomer of a copolymer of ethylene and α-olefin having 3 to 20 carbon atoms such as propene, 1-butene, 4-methyl-pentene-1, hexene, octene, decene, or the like, and specific examples thereof include Tafmer (Mitsui Chemical Co.), Engage (Dow Chemical Company), and the like.

In the medical tube according to the present invention, it is preferable that the cross-copolymerized olefin-aromatic vinyl compound-diene copolymer is added at a content of 2 to 30 parts by weight, preferably, 5 to 25 parts by weight to a mixture of 100 parts by weight of the polypropylene based elastomer having Shore hardness of 35A or more but 100A or less and 5 to 50 parts by weight of the polypropylene resin or styrene based elastomer. In the above-mentioned range, a synergy expression effect as the polymer based compatibilizer may be excellent, excellent kink resistance and toughness may be secured, there is no risk that kink resistance and transparency will be damaged.

It is preferable that the polypropylene based elastomer according to the present invention has Shore hardness of 35A or more but 100A or less. In the case in which the Shore hardness is less than 35A, the polypropylene based elastomer is excessively flexible, such that it is difficult to secure the desired rigidity, and in the case in which the Shore hardness is more than 100A, flexibility is insufficient, such that it is also difficult to secure the desired rigidity.

In the present invention, the polypropylene resin is a polypropylene homopolymer or a polypropylene random copolymer or polypropylene block copolymer prepared by copolymerization of propylene as a main ingredient and α-olefin having 2 to 20 carbon atoms such as ethylene, 1-butene, 4-methyl-pentene-1, hexene, octene, decene, or the like. Among them, in consideration of transparency and compatibility with the polypropylene based elastomer, it is preferable to use the polypropylene random copolymer.

It is preferable that an addition amount of the polypropylene resin according to the present invention is 5 to 50 parts by weight, preferably 10 to 40 parts by weight based on 100 parts by weight of the polypropylene based elastomer having Shore hardness of 35A or more but 100A or less. In the case in which the addition amount is less than 5 parts by weight, strength may be excessively decreased, and in the case in which the addition amount is more than 50 parts by weight, kink resistance may be deteriorated.

In the present invention, styrene based elastomer is a copolymer elastomer containing styrene, and specific examples thereof may include a styrene-butadiene block copolymer elastomer, a hydrogenated styrene-butadiene elastomer, a styrene-isoprene block copolymer elastomer, a styrene-butadiene-styrene block copolymer elastomer, a styrene-isoprene-styrene block copolymer elastomer, a styrene-butadiene-butylene-styrene block copolymer elastomer, a styrene-ethylene-butylene-styrene block copolymer elastomer, a styrene-ethylene-propylene-styrene block copolymer elastomer, and the like.

It is preferable that an addition amount of the styrene based elastomer according to the present invention is 5 to 50 parts by weight, preferably 10 to 40 parts by weight based on 100 parts by weight of the polypropylene based elastomer having Shore hardness of 35A or more but 100A or less. In the case in which the addition amount is less than 5 parts by weight, flexibility may be excessively deteriorated, and in the case in which the addition amount is more than 50 parts by weight, strength may be significantly decreased.

The medical tube according to the present invention is a tube having at least one layer, and the desired object may be achieved by a tube having a single layer structure, but in the case of manufacturing a tube having a multilayer structure composed of two layers, three layers, or the like, the desired object may be further effectively achieved.

For example, a tube having a two layer structure composed of one layer made of the cross-copolymerized olefin-aromatic vinyl compound-diene copolymer having Shore hardness of 40A or more but 100A or less or the resin composition containing the copolymer and the other layer made of syndiotactic 1,2-polybutadiene, the polyethylene based elastomer, or the mixture thereof may be considered. Further, a tube having a three layer structure composed of external, intermediate, and internal layers, that is, a tube having a three layer structure composed of internal and external layers made of the cross-copolymerized olefin-aromatic vinyl compound-diene copolymer having Shore hardness of 40A or more but 100A or less or the resin composition containing the copolymer and an intermediate layer made of syndiotactic 1,2-polybutadiene, the polyethylene based elastomer, or the mixture thereof, a tube having a three layer structure composed of an intermediate layer made of the cross-copolymerized olefin-aromatic vinyl compound-diene copolymer having Shore hardness of 40A or more but 100A or less or the resin composition containing the copolymer and internal and external layers made of syndiotactic 1,2-polybutadiene, the polyethylene based elastomer, or the mixture thereof, and the like, may be considered.

However, in the case of the tube having the multilayer structure as described above, when a proportion of the layer made of the cross-copolymerized olefin-aromatic vinyl compound-diene copolymer having Shore hardness of 40A or more but 100A or less or the resin composition containing the copolymer is at least 50% or more, the desired toughness may be secured. The proportion is preferably 60% or more, and more preferably, 70% or more.

Alternatively, a tube having a two layer structure composed of one layer containing the polypropylene based elastomer having Shore hardness of 35A or more but 100A or less and the other layer made of a composition obtained by adding the cross-copolymerized olefin-aromatic vinyl compound-diene copolymer to the mixture of the polypropylene based elastomer having Shore hardness of 35A or more but 100A or less and the polypropylene resin or styrene based elastomer may be considered. Further, a tube having a three layer structure composed of external, intermediate, and internal layers, for example, a tube having a three layer structure composed of internal and external layers containing the polypropylene based elastomer having Shore hardness of 35A or more but 100A or less and an intermediate layer made of the polypropylene based elastomer having Shore hardness of 35A or more but 100A or less, the polypropylene resin, and the cross-copolymerized olefin-aromatic vinyl compound-diene copolymer, a tube having a three layer structure composed of an intermediate layer containing the polypropylene based elastomer having Shore hardness of 35A or more but 100A or less and internal and external layers made of the polypropylene based elastomer having Shore hardness of 35A or more but 100A or less, the polypropylene resin, and the cross-copolymerized olefin-aromatic vinyl compound-diene copolymer, a tube having a three layer structure composed of internal and external layers containing the polypropylene based elastomer having Shore hardness of 35A or more but 100A or less and an intermediate layer made of the polypropylene based elastomer having Shore hardness of 35A or more but 100A or less, the styrene based elastomer, and the cross-copolymerized olefin-aromatic vinyl compound-diene copolymer, a tube having a three layer structure composed of an intermediate layer containing the polypropylene based elastomer having Shore hardness of 35A or more but 100A or less and internal and external layers made of the polypropylene based elastomer having Shore hardness of 35A or more but 100A or less, the styrene based elastomer, and the cross-copolymerized olefin-aromatic vinyl compound-diene copolymer, and the like, may be considered.

However, in the case of this tube having the multilayer structure, similarly to the above-mentioned case, a proportion of the layer made of the composition obtained by adding the cross-copolymerized olefin-aromatic vinyl compound-diene copolymer to the mixture of the polypropylene based elastomer having Shore hardness of 35A or more but 100A or less and the polypropylene resin or styrene based elastomer needs to be at least 50% or more, preferably 60% or more, and more preferably 70% or more. In the case in which the proportion satisfies the above-mentioned content, it is easy to secure the desired kink resistance and toughness.

The mixture of the synthetic resin used in the present invention may be dry blended in a pellet state or be kneaded by a single-screw extruder, a twin-screw extruder, a mixing roll, a Banbury mixer, a kneader, or the like. In addition, general additives, for example, an antioxidant, a heat stabilizer, a UV stabilizer, a lubricant, a processing aide, an anti-blocking agent, or the like, may be mixed in a range in which the object of the present invention is not damaged.

Further, in the case of the medical supplies mainly composed of a tube, for example, the infusion set, the blood transfusion set, a medical liquid container set, the medical catheter, or the like, in order to complete perfectly PVC-free medical supplies, a measure for some articles injection-molded using PVC as well as the tube has been required. For example, an infusion set is composed of a tube, a drip chamber, a clamp, a spike, a needle, a rubber tube, an air trap, and the like, but a tube and a drip chamber according to the related art are manufactured using flexible PVC, such that the drip chamber in addition to the tube need to be developed as PVC-free products.

In the case of injection-molding the cross-copolymerized olefin-aromatic vinyl compound-diene copolymer resin having Shore hardness of 40A or more but 100A or less according to the present invention or the composition containing the resin, for example, the mixture of the cross-copolymerized olefin-aromatic vinyl compound-diene copolymer having Shore hardness of 40A or more but 100A or less and syndiotactic 1,2-polybutadiene, and/or the polyethylene based elastomer, etc., using an injection-molding machine to thereby obtain medical molded articles such as the drip chamber of the infusion set, the medical molded article may be easily combined with the medical tube according to the present invention, thereby making it possible to manufacture a perfect PVC-free medical supply.

Further, in the case of injection-molding a composition obtained by adding 2 to 30 parts by weight of the cross-copolymerized olefin-aromatic vinyl compound-diene copolymer to a mixture of 100 parts by weight of the polypropylene based elastomer having Shore hardness of 35A or more but 100A or less and 5 to 50 parts by weight of the polypropylene resin or a composition obtained by adding 2 to 30 parts by weight of the cross-copolymerized olefin-aromatic vinyl compound-diene copolymer to a mixture of 100 parts by weight of the polypropylene based elastomer having Shore hardness of 35A or more but 100A or less and 5 to 50 parts by weight of the styrene based elastomer using a general injection-molding machine to thereby obtain medical molded articles such as the drip chamber of the infusion set, the medical molded article may be easily combined with the medical tube according to the present invention, thereby making it possible to manufacture a perfect PVC-free medical supply.

As a combination method of the tube according to the present invention and molded articles, in the case of processing a combination part of the tube with an organic solvent such as cyclohexanone, tetrahydrofuran, cyclohexane, methylethylketone, acetone, ethyl acetate, or the like, the tube and the molded articles are easily adhered to and combined with each other. Further, in the case of combining the tube with a molded article injection-molded using a synthetic resin having a polarity, for example, a polycarbonate resin, an acrylonitrile-butadiene-styrene (ABS) resin, a polyester resin, a polyamide resin, or the like, in consideration that the tube and the molded articles according to the present invention are non-polar, when a combination part is subjected to corona discharge treatment, ozone treatment, electron beam treatment, plasma discharge treatment, UV laser treatment, chemical treatment, or the like, so as to have a polarity and then treated with an organic solvent, the tube and the molded articles may be easily adhered and combined with each other.

### [Advantageous Effects]

A tube having a single layer structure or multilayer structure containing a cross-copolymerized olefin-aromatic vinyl-diene copolymer having Shore hardness of 40A or more but 100A or less as a main ingredient has excellent kink resistance, toughness, transparency, and non-adsorption of drugs, etc., and made of a non-toxic PVC-free material, such that the tube may be combined with molded articles injection-molded using the resin to thereby be usefully used as medical supplies such as an infusion set, a blood transfusion set, a medical liquid container set, a medical catheter, and the like.

### [Description of Drawings]

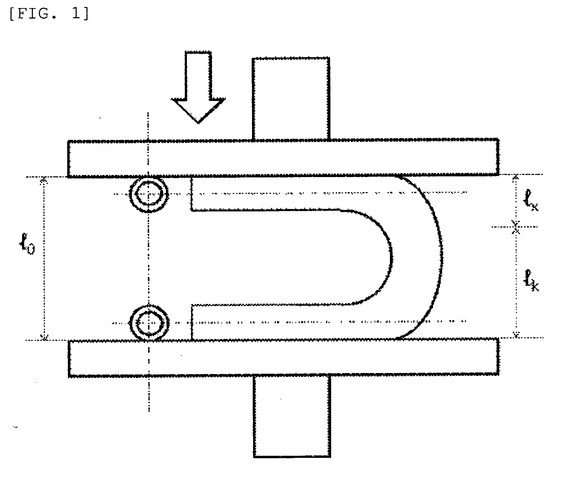

FIG. 1 shows a form in which a sample tube is mounted in a jig in order to evaluate kink resistance of the tube.

### [Detailed Description of Main Elements]

ℓ₀: initial position (30mm)
ℓₓ: compression distance (at maximum test force)
ℓₖ: kink distance

### [Best Mode]

Hereinafter, Examples will be provided in order to describe the present invention in more detail, but the present invention is not limited to the following Examples. In addition, unless otherwise specified, the processing in each of the following Examples and Comparative Examples is performed in the same manner in Example 1.

Kink resistance, toughness, transparency, and non-adsorption of drugs of sample tubes manufactured according to the following Examples and Comparative Examples were evaluated as follows.

### (Kink Resistance)

After both ends of a sample tube cut at a length of 120 mm were fixed to a jig prepared in advance as shown in FIG. 1, a kink distance (ℓₖ) at which kink occurred while compressing at a rate of 20 mm/min in a universal testing machine (UTM, Tinius Olsen) was measured. The kink distance was calculated by subtracting a compression distance (at maximum test force, ℓₓ) from an initial position (ℓ₀, 30 mm). The shorter the kink distance, the more excellent kink resistance, and kink resistance was evaluated in 4 grades as shown in Table 1.

**[Table 1]**

| Classification | ⊚(Excellent) | ○(Good) | Δ(Fair) | X(Poor) |
|---|---|---|---|---|
| Kink Distance (ℓₖ , mm) | less than 10 | 10 or more but less than 15 | 15 or more but less than 20 | 20 or more |

### (Toughness)

Toughness of the tube was evaluated as tensile strength and elongation at the time of fracture. That is, tensile strength (MPa) and elongation (%) at the time of fracture of the sample were measured at a tensile speed of 200 mm/min and a temperature of 23°C using a universal testing machine (UTM, Tinius Olsen), and as shown in Table 2, the results were evaluated in 4 grades. In addition, overall evaluation of toughness was determined by the lowest of evaluation values of tensile strength and elongation. (For example, when tensile strength was ⊚ (excellent) and elongation was X (poor), the overall evaluation was X (poor).)

**[Table 2]**

| Classification | ⊚(Excellent) | ○(Good) | Δ(Fair) | X(Poor) |
|---|---|---|---|---|
| Tensile Strength(MPa) | 20 or more | 15 or more but less than 20 | 10 or more but less than 15 | less than 10 |
| Elongation (%) | 700 or more | 600 or more but less than 700 | 500 or more but less than 600 | less than 500 |

### (Transparency)

Transparency of the tube was evaluated in 4 grades as shown in Table 3 on the basis of Haze (%) measured using a haze meter (Toyoseiki Corp.) according to ASTM D1003 after cutting the sample tube at a significantly small size and forming a sheet having a thickness of 0.6 mm in a hot press.

**[Table 3]**

| Classification | ⊚(Excellent) | ○(Good) | Δ(Fair) | X(Poor) |
|---|---|---|---|---|
| Haze(%) | less than 10 | 10 or more but less than 20 | 20 or more but less than 40 | 40 or more |

### (Non-adsorption of drug)

Non-adsorption of drugs was evaluated as non-adsorption of nitroglycerin. First, 60ml of nitroglycerin injection (effective ingredient: 50mg/100ml, JW Pharmaceutical Corp.) was slowly injected into 1L of normal saline (JW Pharmaceutical Corp.) and slowly stirred. Instantly, the nitroglycerin containing normal saline was sampled in an injection syringe equipped with an injection needle, and a concentration of this solution was set as a blank concentration (C₀). A sample tube mounted in an infusion set was pressed and closed by a flow controlling clamp, and a rubber stopper of a normal saline container was penetrated using a needle of a drip chamber. The drip chamber was pumped, such that the drip chamber was about half filled with normal saline from the bottom. After filling normal saline in the sample tube by slowly loosening the flow controlling clamp, the sample tube was attached to an infusion pump (Nissho Corp, FP-2001). A flow rate was set to 40 ml/hr, the flow controlling clamp was completely loosened, and infusion was initiated by pushing an initiation switch. Normal saline flowed from an end of the sample tube was received in a measuring cylinder, sampling was performed at each predetermined interval, and a concentration was measured by high pressure liquid chromatography (HPLC, Shiseido) (column: CAPCELL PAK SG120, temperature: 30°C, moving phase: methanol/water=11/9, detector UV wavelength: 210 nm, flow rate: 0.8 mℓ/min). Infusion was performed for 3 hours, and the sampling was performed every 10 minutes, such that a concentration change behavior was observed, and a concentration change rate [(C₁ - C₀)/C₀ x 100(%)] was calculated from a final concentration after 3 hours (C₁) and the blank concentration (C₀). When concentration of nitroglycerin was decreased, it was judged that nitroglycerin was adsorbed in the sample tube corresponding to a decreased concentration, and non-adsorption of the drug was evaluated in 4 grades as shown in Table 4.

**[Table 4]**

| Classification | ⊚(Excellent) | ○(Good) | Δ(Fair) | X(Poor) |
|---|---|---|---|---|
| Nitroglycerin Concentration Change Rate (%) after 3 Hours | less than 2 | 2 or more but less than 5 | 5 or more but less than 10 | 10 or more |

### (Solvent adhesion)

The tube sample and a drip chamber sample were adhered to each other using cyclohexane as a solvent. Solvent adhesion was judged by observing whether or not the tube was separated from the drip chamber in the case of strongly pulling the tube after adhesion. When the tube was not separated, solvent adhesion was judged as good (○), and when the tube was separated, solvent adhesion was judged as poor (X).

### (EOG sterilization resistance)

After sterilizing an infusion set equipped with the sample tube and the drip chamber using ethylene oxide gas (EOG) (at 60°C for 2 hours), when shapes of the sample tube and the drip chamber did not change, EOG sterilization resistance was judged as good (○), and if the shapes changed just a little bit, EOG sterilization resistance was judged as poor (X).

### [Example 1]

First, a cross-copolymerized olefin-aromatic vinyl compound-diene copolymer resin (Electro-Chemical Industry Co., SE-based flexible resin, Grade AC081, SE-A) having Shore hardness of 63A and a melt index (200°C, 10 kg) of 20 (g/10min) was prepared and syndiotactic 1,2-polybutadiene (JSR, RB810, RB-A) having Shore hardness of 79A, a crystallinity of 18%, and a melt index (150°C, 2.16 kg) of 3 g/10min was prepared. A composition pellet in which 20 parts by weight of RB-A was mixed based on 100 parts by weight of the SE-A resin was injected into a hopper, and extruded and molded in an extruder (cylinder temperature: 120/130/140°C, die temperature: 140°C), thereby manufacturing a tube sample (T-1) having an inner diameter of 2.1 mm and an outer diameter of 3.4 mm. Kink resistance, toughness, transparency, and non-adsorption of drug of the tube sample were evaluated, and the results were shown in Table 5.

### [Example 2]

Syndiotactic 1,2-polybutadiene (JSR, RB820, RB-B) having Shore hardness of 95A, a crystallinity of 25%, and a melt index (150°C, 2.16 kg) of 3 g/10min was prepared. A tube sample (T-2) having an inner diameter of 2.1 mm and an outer diameter of 3.4 mm was manufactured in the same manner as in Example 1 except for using a composition in which 30 parts by weight of RB-A was mixed based on 100 parts by weight of the SE-A resin. Kink resistance, toughness, transparency, and non-adsorption of the drug of the tube sample were evaluated, and the results were shown in Table 5.

### [Example 3]

A polyethylene based elastomer (Dow Chemical Company, Engage EG8200, PEE-A) having Shore hardness of 66A and a melt index (190°C, 2.16 kg) of 5 (g/10min) was prepared. A tube sample (T-3) having an inner diameter of 2.1 mm and an outer diameter of 3.4 mm was manufactured in the same manner as in Example 1 except for using a composition in which 40 parts by weight of PEE-A was mixed based on 100 parts by weight of the SE-A resin. Kink resistance, toughness, transparency, and non-adsorption of the drug of the tube sample were evaluated, and the results were shown in Table 5.

### [Example 4]

A cross-copolymerized olefin-aromatic vinyl compound-diene copolymer resin (Electro-Chemical Industry Co., SE-based flexible resin, Grade ACP2520, SE-B) having Shore hardness of 84A and a melt index (200°C, 10 kg) of 20 (g/10min) was prepared. A tube sample (T-4) having an inner diameter of 2.1 mm and an outer diameter of 3.4 mm was manufactured in the same manner as,in Example 1 except for using a composition in which 150 parts by weight of RB-A was mixed based on 100 parts by weight of the SE-A resin. Kink resistance, toughness, transparency, and non-adsorption of the drug of the tube sample were evaluated, and the results were shown in Table 5.

### [Example 5]

A tube sample (T-5) having an inner diameter of 2.1 mm and an outer diameter of 3.4 mm was manufactured in the same manner as in Example 1 except for using a composition in which 20 parts by weight of RB-A, 20 parts by weight of PEE-A were mixed with each other based on 100 parts by weight of the SE-A resin. Kink resistance, toughness, transparency, and non-adsorption of the drug of the tube sample were evaluated, and the results were shown in Table 5.

### [Comparative example 1]

Kink resistance, toughness, transparency, and non-adsorption of the drug were evaluated in the same manner as in Example 1 using an infusion set (Shinchang Medical) using a commercialized flexible PVC based tube, and the flexible PVC based tube (T-C1) separated from this infusion set, and the results were shown in Table 5.

### [Comparative example 2]

A tube sample (T-C2) having an inner diameter of 2.1 mm and an outer diameter of 3.4 mm was manufactured in the same manner as in Example 1 except for using only 100 parts by weight of RB-A. Kink resistance, toughness, transparency, and non-adsorption of the drug of the tube sample were evaluated, and the results were shown in Table 5.

### [Comparative example 3]

A polypropylene copolymer resin (Korea Petrochemical Ind. Co., Polypro F8308, PP-A) having hardness of 70R, a melting point of 130°C, a melt index (230°C, 2.16 kg) of 7.5 g/10min and a polypropylene based elastomer (ExxonMobil, Vistamaxx 3,000, PPE-A) having Shore hardness of 86A and a melt index (230°C, 2.16 kg) of 7(g/10min) were prepared. A tube sample (T-C3) having an inner diameter of 2.1 mm and an outer diameter of 3.4 mm was manufactured in the same manner as in Example 1 except for using a composition in which 150 parts by weight of PPE-A was mixed based on 100 parts by weight of the PP-A resin. Kink resistance, toughness, transparency, and non-adsorption of the drug of the tube sample were evaluated, and the results were shown in Table 5.

### [Comparative example 4]

A polystyrene-isoprene copolymer based elastomer (Kuraray, Hybrar 7125, PSE-A) having hardness of 64A and a melt index (230°C, 2.16 kg) of 5.7(g/10min) was prepared. First, a tube sample (T-C4) having an inner diameter of 2.1 mm and an outer diameter of 3.4 mm was manufactured in the same manner as in Example 1 except for using a composition in which 200 parts by weight of PSE-A was mixed based on 100 parts by weight of the PP-A resin. Kink resistance, toughness, transparency, and non-adsorption of the drug of the tube sample were evaluated, and the results were shown in Table 5.

### [Comparative example 5]

A tube sample (T-C5) having an inner diameter of 2.1 mm and an outer diameter of 3.4 mm was manufactured in the same manner as in Example 1 except for using a composition in which 100 parts by weight of PSE-A was mixed based on 100 parts by weight of the RB-A resin. Kink resistance, toughness, transparency, and non-adsorption of the drug of the tube sample were evaluated, and the results were shown in Table 5.

**[Table 5]**

| **Classification** | **Single Layer Tube Composition (Part by Weight)** | **Kink Resistance** | | **Toughness** | | | **Transparency** | | **Non-Adsorption of Drug** | |
|---|---|---|---|---|---|---|---|---|---|---|
| | | Kink Distance (mm) | Overall Evaluation | Tensile Strength (MPa) | Elongation (%) | Overall Evaluation | Haze (%) | Overall Evaluation | Concentration Change Rate (%) | Overall Evaluation |
| **Example** 1 | SE-A 100 | 5 | ⊚ | 19 | 850 | ○ | 5 | ⊚ | 1.6 | ⊚ |
| | RB-A 20 | | | | | | | | | |
| **Example** 2 | SE-A 100 | 7 | ⊚ | 21 | 770 | ⊚ | 4 | ⊚ | 1.4 | ⊚ |
| | RB-B 30 | | | | | | | | | |
| **Example** 3 | SE-A 100 | 6 | ⊚ | 17 | 910 | ○ | 6 | ⊚ | 1.4 | ⊚ |
| | PEE-A 40 | | | | | | | | | |
| **Example** 4 | SE-B 100 | 9 | ⊚ | 24 | 710 | ⊚ | 4 | ⊚ | 1.2 | ⊚ |
| | RB-B 25 | | | | | | | | | |
| **Example** 5 | SE-A 100 | 9 | ⊚ | 21 | 730 | ⊚ | 6 | ⊚ | 1.3 | ⊚ |
| | RB-A 20 | | | | | | | | | |
| | PPE-A 20 | | | | | | | | | |
| **Comparative Example 1** | PVC-A 100 | 7 | ⊚ | 16 | 710 | ○ | 9 | ⊚ | 19.1 | X |
| **Comparative Example 2** | RB-A 100 | 12 | ○ | 6 | 750 | X | 7 | ⊚ | 0.5 | ⊚ |
| **Comparative** | PP-A 100 | 22 | X | 27 | 590 | Δ | 8 | ⊚ | 0.9 | ⊚ |
| **Example 3** | PPE-A 150 | | | | | | | | | |
| **Comparative Example 4** | PP-A 100 | 24 | X | 12 | 610 | Δ | 9 | ⊚ | 1.8 | ⊚ |
| | PSE-A 200 | | | | | | | | | |
| **Comparative Example 5** | RB-A 100 | 13 | ○ | 7 | 690 | X | 11 | ○ | 2.1 | ○ |
| | PSE-A 100 | | | | | | | | | |

### [Example 6]

The SE-B, RB-A, and RB-B resins were prepared. A tube sample (T-6) having a two layer structure, an inner diameter of 2.1 mm, and an outer diameter of 3.4 mm was manufactured in the same manner as in Example 1 except for using only the SE-B resin as a raw material for an external layer, using a composition in which 100 parts by weight of RB-A and 50 parts by weight of RB-B were mixed with each other as a raw material for an internal layer, and adjusting an external/internal layer thickness configuration (%) to 60/40 in a multilayer extruder (See Table 6). Kink resistance, toughness, transparency, and non-adsorption of the drug of the tube sample were evaluated, and the results were shown in Table 7.

### [Example 7]

A tube sample (T-7) having a two layer structure, an inner diameter of 2.1 mm, and an outer diameter of 3.4 mm was manufactured in the same manner as in Example 1 except for using a composition in which 100 parts by weight of RB-A and 50 parts by weight of RB-B were mixed with each other as a raw material for an external layer, using a composition in which 100 parts by weight of SE-A and 20 parts by weight of RB-B were mixed with each other as a raw material for an internal layer, and adjusting an external/internal layer thickness configuration (%) to 20/80 in a multilayer extruder (See Table 6). Kink resistance, toughness, transparency, and non-adsorption of the drug of the tube sample were evaluated, and the results were shown in Table 7.

### [Example 8]

A tube sample (T-8) having a three layer structure, an inner diameter of 2.1 mm, and an outer diameter of 3.4 mm was manufactured in the same manner as in Example 1 except for using a composition in which 100 parts by weight of RB-A and 50 parts by weight of RB-B were mixed with each other as a raw material for external and internal layers, using only the SE-B resin as a raw material for an intermediate layer, and adjusting an external/intermediate/internal layer thickness configuration (%) to 20/60/20 in a multilayer extruder (See Table 6). Kink resistance, toughness, transparency, and non-adsorption of the drug of the tube sample were evaluated, and the results were shown in Table 7.

### [Example 9]

A tube sample (T-9) having a three layer structure, an inner diameter of 2.1 mm, and an outer diameter of 3.4 mm was manufactured in the same manner as in Example 1 except for using only the SE-A resin as a raw material for external and internal layers, using only the PEE-A resin as a raw material for an intermediate layer, and adjusting an external/intermediate/internal layer thickness configuration (%) to 30/40/30 in a multilayer extruder (See Table 6). Kink resistance, toughness, transparency, and non-adsorption of the drug of the tube sample were evaluated, and the results were shown in Table 7.

### [Comparative example 6]

First, linear low density polyethylene (SK Energy, LLDPE FT850, PE-A) having a softening point of 102°C and a melt index (190°C, 2.16 kg) of 3.5 (g/10min) was prepared. A tube sample (T-C6) having a two layer structure, an inner diameter of 2.1 mm, and an outer diameter of 3.4 mm was manufactured in the same manner as in Example 1 except for using a composition in which 100 parts by weight of RB-A and 100 parts by weight of PSE-A were mixed with each other as a raw material for an external layer, using only PE-A as a raw material for an internal layer, and adjusting an external/internal layer thickness configuration (%) to 85/15 in a multilayer extruder (See Table 6). Kink resistance, toughness, transparency, and non-adsorption of the drug of the tube sample were evaluated, and the results were shown in Table 7.

### [Comparative example 7]

A tube sample (T-C7) having a three layer structure, an inner diameter of 2.1 mm, and an outer diameter of 3.4 mm was manufactured in the same manner as in Example 1 except for using only the RB-A resin as a raw material for external and internal layers, using only the PE-A resin as a raw material for an intermediate layer, and adjusting an external/intermediate/internal layer thickness configuration (%) to 45/10/45 in a multilayer extruder (See Table 6). Kink resistance, toughness, transparency, and non-adsorption of the drug of the tube sample were evaluated, and the results were shown in Table 7.

**[Table 6]**

| **Classification** | **Multilayer Tube Sample Name** | **Layer Composition in Multilayer Tube (Part by Weight)** | | | **External/intermediate/ Internal Layer Thickness Configuration (%)** |
|---|---|---|---|---|---|
| | | **External Layer** | **Intermediate Layer** | **Internal Layer** | |
| **Example** 6 | T-6 | SE-B 100 | - | RB-A 100 | 60/0/40 |
| | | | | RB-B 50 | |
| **Example** 7 | T-7 | RB-A 100 | - | SE-A 100 | 20/0/80 |
| | | RB-B 50 | | RB-B 20 | |
| **Example** 8 | T-8 | RB-A 100 | SE-B 100 | RB-A 100 | 20/60/20 |
| | | RB-B 50 | | RB-B 50 | |
| **Example** 9 | T-9 | SE-A 100 | PPE-A 100 | SE-A 100 | 30/40/30 |
| **Comparative Example** 6 | T-C6 | RB-A 100 | - | PE-A 100 | 85/0/15 |
| | | PSE-A 100 | | | |
| **Comparative Example** 7 | T-C7 | RB-A 100 | PE-A 100 | RB-A 100 | 45/10/45 |

**[Table 7]**

| **Classification** | **Tube Sample Name** | **Kink Resistance** | | **Toughness** | | | **Transparency** | | **Non-Adsorption of Drug** | |
|---|---|---|---|---|---|---|---|---|---|---|
| | | Kink Distance (mm) | Overall Evaluation | Tensile Strength (MPa) | Elongation (%) | Overall Evaluation | Haze (%) | Overall Evaluation | Concentration Change Rate (%) | Overall |
| **Example** 6 | T-6 | 8 | ⊚ | 20 | 810 | ⊚ | 4 | ⊚ | 0.7 | ⊚ |
| **Example** 7 | T-7 | 9 | ⊚ | 21 | 790 | ⊚ | 5 | ⊚ | 0.9 | ⊚ |
| **Example** 8 | T-8 | 4 | ⊚ | 18 | 820 | ○ | 4 | ⊚ | 0.5 | ⊚ |
| **Example** 9 | T-9 | 12 | ○ | 23 | 740 | ⊚ | 6 | ⊚ | 1.0 | ⊚ |
| **Comparative Example 6** | T-C6 | 23 | X | 14 | 610 | Δ | 18 | ○ | 0.8 | ⊚ |
| **Comparative Example 7** | T-C7 | 20 | X | 10 | 680 | Δ | 19 | ○ | 0.6 | ⊚ |

### [Example 10]

A drip chamber sample (DC-1) was manufactured so that a distance from an end of a drop pipette to an end of the drip chamber was 50 mm by injection-molding a composition in which 40 parts by weight of RB-A was mixed based on 100 parts by weight of SE-A using an injection machine. An infusion set was manufactured by adhering the drip chamber sample (DC-1) and the tube sample (T-1) to each other using cyclohexane as a solvent and connecting the existing connection tubes and injection molded articles according to the related art. Solvent adhesion and EOG sterilization resistance of the infusion set after being sterilized (60°C, 2 hours) using EOG were evaluated, and the results were shown in Table 8.

### [Example 11]

An infusion set was manufactured in the same manner in Example 10 except for manufacturing a drip chamber sample (DC-2) by injection-molding a composition in which 20 parts by weight of PEE-A based on 100 parts by weight of SE-A. Solvent adhesion and EOG sterilization resistance were evaluated, and the results were shown in Table 8.

**[Table 8]**

| **Classification** | **Used Drip Chamber** | **Used Tube** | **Solvent Adhesion** | **EOG Sterilization Resistance** |
|---|---|---|---|---|
| **Example** 10 | DC-1 | T-1 | ○ | ○ |
| **Example** 11 | DC-2 | T-1 | ○ | ○ |

### [Example 12]

A polypropylene based elastomer (ExxonMobil, Vistamaxx 3,000, PPE-A) having Shore hardness of 86A and a melt index (230°C, 2.16 kg) of 7 (g/10min), a polypropylene copolymer resin (Korea Petrochemical Ind. Co., Polypro F8308, PP-A) having hardness of 70R, a melting point of 130°C, a melt index (230°C, 2.16 kg) of 7.5 (g/10min), and a cross-copolymerized olefin-aromatic vinyl compound-diene copolymer resin (Electro-Chemical Industry Co., SE-based flexible resin, Grade AC081, SE-A) having Shore hardness of 63A and a melt index (200°C, 10 kg) of 20 (g/10min) were prepared. A composition pellet in which 100 parts by weight of PPE-A, 20 parts by weight of PP-A, and 5 parts by weight of SE-A were mixed with each other was injected into a hopper, and extruded and molded in an extruder (cylinder temperature: 150/160/170°C, die temperature: 170°C), thereby manufacturing a tube sample (T-1) having an inner diameter of 2.1 mm and an outer diameter of 3.4 mm. Kink resistance, toughness, transparency, and non-adsorption of the drug of the tube sample were evaluated, and the results were shown in Table 9.

### [Example 13]

A tube sample (T-2) having an inner diameter of 2.1 mm and an outer diameter of 3.4 mm was manufactured in the same manner as in Example 12 except for using a composition in which 100 parts by weight of PPE-A, 20 parts by weight of PP-A, and 10 parts by weight of SE-A were mixed with each other. Kink resistance, toughness, transparency, and non-adsorption of the drug of the tube sample were evaluated, and the results were shown in Table 9.

### [Example 14]

A tube sample (T-3) having an inner diameter of 2.1 mm and an outer diameter of 3.4 mm was manufactured in the same manner as in Example 12 except for using a composition in which 100 parts by weight of PPE-A, 30 parts by weight of PP-A, and 25 parts by weight of SE-A were mixed with each other. Kink resistance, toughness, transparency, and non-adsorption of the drug of the tube sample were evaluated, and the results were shown in Table 9.

### [Example 15]

A polypropylene based elastomer (ExxonMobil, Vistamaxx 3,980FL, PPE-B) having Shore hardness of 89A and a melt index (230°C, 2.16 kg) of 3.6 (g/10min) and a polystyrene-isoprene copolymer based elastomer (Kuraray, Hybrar 7125, PSE-A) having hardness of 64A and a melt index (230°C, 2.16 kg) of 5.7(g/10min) were prepared, and a cross-copolymerized olefin-aromatic vinyl compound-diene copolymer resin (Electro-Chemical Industry Co., SE-based flexible resin, Grade ACP2520, SE-B) having Shore hardness of 84A and a melt index (200°C, 10 kg) of 20 (g/10min) was also prepared. A tube sample (T-4) having an inner diameter of 2.1 mm and an outer diameter of 3.4 mm was manufactured in the same manner as in Example 12 except for using a composition in which 100 parts by weight of PPE-B, 20 parts by weight of PSE-A, and 10 parts by weight of SE-B were mixed with each other. Kink resistance, toughness, transparency, and non-adsorption of the drug of the tube sample were evaluated, and the results were shown in Table 9.

### [Example 16]

A tube sample (T-5) having an inner diameter of 2.1 mm and an outer diameter of 3.4 mm was manufactured in the same manner as in Example 12 except for using a composition in which 100 parts by weight of PPE-B, 30 parts by weight of PSE-A, and 20 parts by weight of SE-A were mixed with each other. Kink resistance, toughness, transparency, and non-adsorption of the drug of the tube sample were evaluated, and the results were shown in Table 9.

### [Comparative example 8]

Kink resistance, toughness, transparency, and' non-adsorption of the drug were evaluated as the same manner as in Example 12 using an infusion set (Shinchang Medical) using a commercialized flexible PVC based tube, and the flexible PVC based tube (T-C1) separated from this infusion set, and the results were shown in Table 9.

### [Comparative example 9]

A tube sample (T-C2) having an inner diameter of 2.1 mm and an outer diameter of 3.4 mm was manufactured in the same manner as in Example 12 except for using a composition in which 100 parts by weight of PPE-A and 30 parts by weight of PP-A were mixed with each other. Kink resistance, toughness, transparency, and non-adsorption of the drug of the tube sample were evaluated, and the results were shown in Table 9.

### [Comparative example 10]

A tube sample (T-C3) having an inner diameter of 2.1 mm and an outer diameter of 3.4 mm was manufactured in the same manner as in Example 12 except for using a composition in which 100 parts by weight of PPE-B and 30 parts by weight of PSE-A were mixed with each other. Kink resistance, toughness, transparency, and non-adsorption of the drug of the tube sample were evaluated, and the results were shown in Table 9.

**[Table 9]**

| **Classification** | **Single Layer Tube Composition (Part by Weight)** | **Kink Resistance** | | **Toughness** | | | **Transparency** | | **Non-Adsorptior of Drug** | |
|---|---|---|---|---|---|---|---|---|---|---|
| | | Kink Distance (mm) | Overall Evaluation | Tensile Strength (MPa) | Elongation (%) | Overall Evaluation | Haze (%) | Overall Evaluation | Concentration Change Rate (%) | Overall Evaluation |
| **Example** 12 | PPE-A 100 | 9 | ⊚ | 19 | 750 | ○ | 7 | ⊚ | 1.6 | ⊚ |
| | PP-A 20 | | | | | | | | | |
| | SE-A 5 | | | | | | | | | |
| **Example** 13 | PPE-A 100 | 8 | ⊚ | 21 | 770 | ⊚ | 6 | ⊚ | 1.4 | ⊚ |
| | PP-A 20 | | | | | | | | | |
| | SE-A 10 | | | | | | | | | |
| **Example** 14 | PPE-A 100 | 7 | ⊚ | 21 | 810 | ⊚ | 5 | ⊚ | 1.4 | ⊚ |
| | PP-A 30 | | | | | | | | | |
| | SE-A 25 | | | | | | | | | |
| **Example** 15 | PPE-B 100 | 9 | ⊚ | 24 | 710 | ⊚ | 4 | ⊚ | 1.2 | ⊚ |
| | PSE-A 20 | | | | | | | | | |
| | SE-B 10 | | | | | | | | | |
| **Example** 16 | PPE-B 100 | 6 | ⊚ | 19 | 730 | ○ | 6 | ⊚ | 1.3 | ⊚ |
| | PSE-A 30 | | | | | | | | | |
| | SE-B 20 | | | | | | | | | |
| **Comparative Example 8** | PVC-A 100 | 7 | ⊚ | 16 | 710 | ○ | 9 | ⊚ | 19.1 | X |
| **Comparative Example 9** | PPE-A 100 | 32 | X | 37 | 510 | Δ | 8 | ⊚ | 0.8 | ⊚ |
| | PP-A 30 | | | | | | | | | |
| **Comparative Example 10** | PPE-B 100 | 7 | ⊚ | 9 | 790 | X | 7 | ⊚ | 1.9 | ⊚ |
| | PSE-A 30 | | | | | | | | | |

### [Example 17]

A tube sample (T-6) having a two layer structure, an inner diameter of 2.1 mm, and an outer diameter of 3.4 mm was manufactured in the same manner as in Example 12 except for using a composition in which 100 parts by weight of PPE-A, 20 parts by weight of PP-A, and 20 parts by weight of SE-A were mixed with each other as a raw material for an external layer, using only PPE-A as a raw material for an internal layer, and adjusting an external/internal layer thickness configuration (%) to 80/20 in a multilayer extruder (See Table 10). Kink resistance, toughness, transparency, and non-adsorption of the drug of the tube sample were evaluated, and the results were shown in Table 11.

### [Example 18]

A tube sample (T-7) having a two layer structure, an inner diameter of 2.1 mm, and an outer diameter of 3.4 mm was manufactured in the same manner as in Example 12 except for using a composition in which 100 parts by weight of PPE-B, 30 parts by weight of PSE-A, and 10 parts by weight of SE-B were mixed with each other as a raw material for an external layer, using a composition in which 100 parts by weight of PPE-A, 20 parts by weight of PP-A, and 10 parts by weight of SE-B were mixed with each other as a raw material for an internal layer, and adjusting an external/internal layer thickness configuration (%) to 70/30 in a multilayer extruder (See Table 10). Kink resistance, toughness, transparency, and non-adsorption of the drug of the tube sample were evaluated, and the results were shown in Table 11.

### [Example 19]

A tube sample (T-8) having a three layer structure, an inner diameter of 2.1 mm, and an outer diameter of 3.4 mm was manufactured in the same manner as in Example 12 except for using only 100 parts by weight of PPE-B as a raw material for external and internal layers, using a composition in which 100 parts by weight of PPE-A, 35 parts by weight of PP-A, and 15 parts by weight of SE-A were mixed with each other as a raw material for an intermediate layer, and adjusting an external/intermediate/internal layer thickness configuration (%) to 10/80/10 in a multilayer extruder (See Table 10). Kink resistance, toughness, transparency, and non-adsorption of the drug of the tube sample were evaluated, and the results were shown in Table 11.

### [Example 20]

A tube sample (T-9) having a three layer structure, an inner diameter of 2.1 mm, and an outer diameter of 3.4 mm was manufactured in the same manner as in Example 12 except for using a composition in which 100 parts by weight of PPE-B, 30 parts by weight of PSE-B, and 10 parts by weight of SE-A were mixed with each other as a raw material for an external layer, using a composition in which 100 parts by weight of PPE-B, 25 parts by weight of PP-A, and 10 parts by weight of SE-A were mixed with each other as a raw material for an intermediate layer, using only PPE-B as a raw material for an internal layer, and adjusting an external/intermediate/internal layer thickness configuration (%) to 20/60/20 in a multilayer extruder (See Table 10). Kink resistance, toughness, transparency, and non-adsorption of the drug of the tube sample were evaluated, and the results were shown in Table 11.

### [Example 21]

A tube sample (T-10) having a three layer structure, an inner diameter of 2.1 mm, and an outer diameter of 3.4 mm was manufactured in the same manner as in Example 12 except for using a composition in which 100 parts by weight of PPE-B, 25 parts by weight of PP-A, and 10 parts by weight of SE-A were mixed with each other as a raw material for an external layer, using only PPE-B as a raw material for an intermediate layer, using a composition in which 100 parts by weight of PPE-B, 25 parts by weight of PP-A, and 10 parts by weight of SE-A were mixed with each other as a raw material for an internal layer, and adjusting an external/intermediate/internal layer thickness configuration (%) to 35/30/35 in a multilayer extruder (See Table 10). Kink resistance, toughness, transparency, and non-adsorption of the drug of the tube sample were evaluated, and the results were shown in Table 11.

### [Comparative example 11]

A tube sample (T-C4) having a two layer structure, an inner diameter of 2.1 mm, and an outer diameter of 3.4 mm was manufactured in the same manner as in Example 12 except for using a composition in which 100 parts by weight of PPE-A and 20 parts by weight of PP-A were mixed with each other as a raw material for an external layer, using only PPE-A as a raw material of an internal layer, and adjusting an external/internal layer thickness configuration (%) to 80/20 in a multilayer extruder (See Table. 10). Kink resistance, toughness, transparency, and non-adsorption of the drug of the tube sample were evaluated, and the results were shown in Table 11.

### [Comparative example 12]

A tube sample (T-C5) having a three layer structure, an inner diameter of 2.1 mm, and an outer diameter of 3.4 mm was manufactured in the same manner as in Example 12 except for using a composition in which 100 parts by weight of PPE-B and 30 parts by weight of PSE-B were mixed with each other as a raw material of an external layer, using a composition in which 100 parts by weight of PPE-B and 25 parts by weight of PP-A were mixed with each other as a raw material for an intermediate layer, using only PPE-B as a raw material for an internal layer, and adjusting an external/intermediate/internal layer thickness configuration (%) to 20/60/20 in a multilayer extruder (See Table 10). Kink resistance, toughness, transparency, and non-adsorption of the drug of the tube sample were evaluated, and the results were shown in Table 11.

**[Table 10]**

| **Classification** | **Multilayer Tube Sample Name** | **Layer Composition in Multilayer Tube (Part by Weight)** | | | **External/Intermediate/ Internal Layer Thickness Configuration (%)** |
|---|---|---|---|---|---|
| | | **External Layer** | **Intermediate Layer** | **Internal Layer** | |
| **Example** 17 | T-6 | PPE-A 100 | - | PPE-A 100 | 80/0/20 |
| | | PP-A 20 | | | |
| | | SE-A 20 | | | |
| **Example** 18 | T-7 | PPE-B 100 | - | PPE-A 100 | 70/0/30 |
| | | PSE-A 30 | | PP-A 20 | |
| | | SE-B 10 | | SE-B 10 | |
| **Example** 19 | T-8 | | PPE-A 100 | PPE-A 100 | 10/80/10 |
| | | PPE-A 100 | PP-A 35 | | |
| | | | SE-A 15 | | |
| **Example** 20 | T-9 | PPE-B 100 | PPE-B 100 | PPE-B 100 | 20/60/20 |
| | | PSE-B 30 | PP-A 25 | | |
| | | SE-A 10 | SE-A 10 | | |
| **Example** 21 | T-10 | PPE-B 100 | | PPE-B 100 | 35/30/35 |
| | | PP-A 25 | PPE-B 100 | PP-A 25 | |
| | | SE-A 10 | | SE-A 10 | |
| **Comparative Example 11** | T-C4 | PPE-A 100 | - | PPE-A 100 | 80/0/20 |
| | | PP-A 20 | | | |
| **Comparative Example 12** | T-C5 | PPE-B 100 | PPE-B 100 | PPE-B 100 | 20/60/20 |
| | | PSE-A 30 | PP-A 25 | | |

**[Table 11]**

| **Classification** | **Tube Sample Name** | **Kink Resistance** | | **Toughness** | | | **Transparency** | | **Non-Adsorption of Drug** | |
|---|---|---|---|---|---|---|---|---|---|---|
| | | **Kink Distance (mm)** | **Overall Evaluation** | **Tensile Strength (MPa)** | **Elongation (%)** | **Overall Evaluation** | **Haze (%)** | **Overall Evaluation** | **Concentration Change Rate (%)** | **Overall Evaluation** |
| **Example** 17 | T-6 | 9 | ⊚ | 23 | 730 | ⊚ | 6 | ⊚ | 1.2 | ⊚ |
| **Example** 18 | T-7 | 9 | ⊚ | 20 | 790 | ⊚ | 5 | ⊚ | 1.1 | ⊚ |
| **Example** 19 | T-8 | 8 | ⊚ | 21 | 750 | ⊚ | 5 | ⊚ | 0.9 | ⊚ |
| **Example** 20 | T-9 | 8 | ⊚ | 22 | 780 | ⊚ | 6 | ⊚ | 1.0 | ⊚ |
| **Example** 21 | T-10 | 6 | ⊚ | 20 | 810 | ⊚ | 4 | ⊚ | 1.0 | ⊚ |
| **Comparative Example 11** | T-C4 | 31 | X | 34 | 490 | X | 11 | ○ | 0.9 | ⊚ |
| **Comparative Example 12** | T-C5 | 22 | X | 29 | 520 | Δ | 12 | ○ | 1,2 | ⊚ |

### [Example 22]

A drip chamber sample (DC-1) was manufactured so that a distance from an end of a drop pipette to an end of the drip chamber was 50 mm by injection-molding a composition in which 100 parts by weight of PPE-A, 20 parts by weight of PP-A, and 10 parts by weight of SE-A were mixed with each other using an injection machine. An infusion set was manufactured by adhering the drip chamber sample (DC-1) and the tube sample (T-1) to each other using cyclohexane as a solvent and connecting the existing connection tubes and injection molded articles according to the related art. Solvent adhesion and EOG sterilization resistance of the infusion set after being sterilized (60°C, 2 hours) using EOG were evaluated, and the results were shown in Table 12.

### [Example 23]

A drip chamber sample (DC-1) was manufactured so that a distance from an end of a drop pipette to an end of the drip chamber was 50 mm by injection-molding a composition in which 100 parts by weight of PPE-B, 20 parts by weight of PSE-A, and 10 parts by weight of SE-A were mixed with each other using an injection machine. An infusion set was manufactured by adhering the drip chamber sample (DC-1) and the tube sample (T-4) to each other using cyclohexane as a solvent and connecting the existing connection tubes and injection molded articles according to the related art. Solvent adhesion and EOG sterilization resistance of the infusion set after being sterilized (60°C, 2 hours) using EOG were evaluated, and the results were shown in Table 12.

**[Table 12]**

| **Classification** | **Used Drip Chamber** | **Used Tube** | **Solvent Adhesion** | **EOG Sterilization Resistance** |
|---|---|---|---|---|
| **Example** 22 | DC-1 | T-1 | ○ | ○ |
| **Example** 23 | DC-2 | T-1 | ○ | ○ |

First, referring to Examples 1 to 5, it may be appreciated that in the case of the tubes having a single layer structure composed of the resin containing the cross-copolymerized olefin-aromatic vinyl-diene copolymer having Shore hardness of 40A or more but 100A or less, these tubes were made of non-toxic PVC-free materials, and kink resistance, toughness, transparency, non-adsorption of drugs, etc. were all excellent, as compared to Comparative Examples 1 to 5, using only flexible PVC according to the related art, using only syndiotactic 1,2-polybutadiene, or using mixtures with other resins. Further, it may be appreciated that in the case of the tubes having a single layer structure composed of the resin containing the composition obtained by adding the cross-copolymerized olefin-aromatic vinyl compound-diene copolymer to the mixture of the polypropylene based elastomer having Shore hardness of 35A or more but 100A or less and the polypropylene resin or styrene based elastomer as in Examples 12 to 16, these tubes were made of non-toxic PVC-free materials, and kink resistance, toughness, transparency, non-adsorption of drugs, etc. were all excellent, as compared to Comparative Examples 8 to 10, using only flexible PVC according to the related art or using the mixture of the polypropylene based elastomer and the polypropylene resin or styrene based elastomer, or the like.

Further, referring to Examples 6 to 9, it may be appreciated that in multilayer films having a layer containing the cross-copolymerized olefin-aromatic vinyl compound-diene copolymer having Shore hardness of 40A or more but 100A or less according to the present invention, kink resistance, toughness, transparency, non-adsorption of drugs, and the like, were also all excellent, as compared to Comparative Examples 6 and 7. In addition, referring to Examples 17 to 21, it may be appreciated that in the case of the tubes having a single layer structure composed of the resin containing the composition obtained by adding the cross-copolymerized olefin-aromatic vinyl compound-diene copolymer to the mixture of the polypropylene based elastomer having Shore hardness of 35A or more but 100A or less and the polypropylene resin or styrene based elastomer, kink resistance, toughness, transparency, non-adsorption of drugs, and the like, were also all excellent, as compared to Comparative Examples 11 and 12.

In addition, referring to Examples 10, 11, 22, and 23, in the case of using the injection-molded articles composed of the resin containing the cross-copolymerized olefin-aromatic vinyl compound-diene copolymer having Shore hardness of 40A or more but 100A or less, solvent adhesion with the tube according to the present invention and EOG sterilization resistance were excellent. When the tube according to the present invention and the molded articles are suitably combined, the resultant article may be effectively used for medical supplies such as an infusion set, a blood transfusion set, a medical liquid container set, a medical catheter, or the like.

## Claims

1. A medical tube comprising at least one layer made of a cross-copolymerized olefin-aromatic vinyl compound-diene copolymer having Shore hardness of 40A or more but 100A or less or a resin composition containing the copolymer.

2. The medical tube of claim 1, wherein the resin composition contains 10 to 200 parts by weight of syndiotactic 1,2-polybutadiene, a polyethylene based elastomer, or a mixture thereof, based on 100 parts by weight of the cross-copolymerized olefin-aromatic vinyl compound-diene copolymer having Shore hardness of 40A or more but 100A or less.

3. The medical tube of claim 1, wherein the resin composition contains 2 to 30 parts by weight of the cross-copolymerized olefin-aromatic vinyl compound-diene copolymer having Shore hardness of 40A or more but 100A or less, based on 100 parts by weight of a polypropylene based elastomer having Shore hardness of 35A or more but 100A or less.

4. The medical tube of claim 3, wherein the resin composition contains 2 to 30 parts by weight of the cross-copolymerized olefin-aromatic vinyl compound-diene copolymer having Shore hardness of 40A or more but 100A or less and 5 to 50 parts by weight of any one selected from polypropylene resins and styrene based elastomers or a mixture thereof, based on 100 parts by weight of the polypropylene based elastomer having Shore hardness of 35A or more but 100A or less.

5. The medical tube of claim 2, wherein it is a tube having a two layer structure composed of one layer made of the cross-copolymerized olefin-aromatic vinyl compound-diene copolymer having Shore hardness of 40A or more but 100A or less or the resin composition containing the copolymer and the other layer made of syndiotactic 1,2-polybutadiene, the polyethylene based elastomer, or the mixture thereof.

6. The medical tube of claim 2, wherein it is a tube having a three layer structure composed of an intermediate layer made of the cross-copolymerized olefin-aromatic vinyl compound-diene copolymer having Shore hardness of 40A or more but 100A or less or the resin composition containing the copolymer, and internal and external layers made of syndiotactic 1,2-polybutadiene, the polyethylene based elastomer, or the mixture thereof.

7. The medical tube of claim 2, wherein it is a tube having a three layer structure composed of internal and external layers made of the cross-copolymerized olefin-aromatic vinyl compound-diene copolymer having Shore hardness of 40A or more but 100A or less or the resin composition containing the copolymer, and an intermediate layer made of syndiotactic 1,2-polybutadiene, the polyethylene based elastomer, or the mixture thereof.

8. The medical tube of claim 3, wherein it is a tube having a two layer structure composed of one layer made of a resin composition containing 5 to 50 parts by weight of a polypropylene resin and 2 to 30 parts by weight of the cross-copolymerized olefin-aromatic vinyl compound-diene copolymer having Shore hardness of 40A or more but 100A or less, based on 100 parts by weight of the polypropylene based elastomer having Shore hardness of 35A or more but 100A or less and the other layer made of the polypropylene based elastomer having Shore hardness of 35A or more but 100A or less.

9. The medical tube of claim 3, wherein it is a tube having a three layer structure composed of an intermediate layer made of a resin composition containing 5 to 50 parts by weight of a polypropylene resin or styrene based elastomer and 2 to 30 parts by weight of the cross-copolymerized olefin-aromatic vinyl compound-diene copolymer having Shore hardness of 40A or more but 100A or less, based on 100 parts by weight of the polypropylene based elastomer having Shore hardness of 35A or more but 100A or less and internal and external layers made of the polypropylene based elastomer having Shore hardness of 35A or more but 100A or less.

10. The medical tube of claim 3, wherein it is a tube having a three layer structure composed of internal and external layers made of a resin composition containing 5 to 50 parts by weight of a polypropylene resin or styrene based elastomer and 2 to 30 parts by weight of the cross-copolymerized olefin-aromatic vinyl compound-diene copolymer having Shore hardness of 40A or more but 100A or less, based on 100 parts by weight of the polypropylene based elastomer having Shore hardness of 35A or more but 100A or less and an intermediate layer made of the polypropylene based elastomer having Shore hardness of 35A or more but 100A or less.

11. The medical tube of claim 1, wherein the cross-copolymerized olefin-aromatic vinyl compound-diene copolymer is obtained by cross-copolymerizing a monomer containing at least 5 mol% of an aromatic vinyl compound with an olefin-aromatic vinyl compound-diene copolymer containing an aromatic vinyl compound in a range of 0.03 mol% or more but 96 mol% or less, diene in a range of 0.0001 mol% or more but 3 mol% or less, and the remainder being olefin.

12. The medical tube of claim 1, wherein a melt index of the cross-copolymerized olefin-aromatic vinyl compound-diene copolymer measured at 200°C under a load of 10 kg is 1 to 50 (g/10min).

13. A medical supply comprising the medical tube of any one of claims 1 to 12, wherein the medical supply is any one of an infusion set, a medical connection line, a port of a medical liquid container, and a medical catheter.
